(19) Europäisches Patentamt
European Patent Office
Office européen des brevets

(11) Numéro de publication : **0 378 014 B1**

# (12) FASCICULE DE BREVET EUROPEEN

(45) Date de publication du fascicule du brevet : **16.09.92 Bulletin 92/38**

(51) Int. Cl.$^5$ : **A61K 7/15**

(21) Numéro de dépôt : **89403017.0**

(22) Date de dépôt : **02.11.89**

## (54) Composition de rasage pour la peau à base de polyorganosiloxanes à fonction hydroxyalkyle et procédé de mise en oeuvre.

Le dossier contient des informations techniques présentées postérieurement au dépôt de la demande et ne figurant pas dans leprésent fascicule.

(30) Priorité : **04.11.88 FR 8814450**

(43) Date de publication de la demande : **18.07.90 Bulletin 90/29**

(45) Mention de la délivrance du brevet : **16.09.92 Bulletin 92/38**

(84) Etats contractants désignés : **AT BE CH DE ES FR GB GR IT LI NL SE**

(56) Documents cités :
**EP-A- 376 820**
**DE-A- 1 467 863**
**FR-A- 2 443 476**
**GB-A- 2 066 659**

(73) Titulaire : **L'OREAL**
**14, Rue Royale**
**F-75008 Paris (FR)**

(72) Inventeur : **Grollier, Jean-François**
**16 bis Boulevard Morland**
**F-75004 Paris (FR)**
Inventeur : **Caudet, Alain**
**255, Boulevard Jean-Jaurès**
**F-92100 Boulogne-Billancourt (FR)**

(74) Mandataire : **Casalonga, Axel et al**
**BUREAU D.A. CASALONGA - JOSSE**
**Morassistrasse 8**
**W-8000 München 5 (DE)**

**EP 0 378 014 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouvelles compositions destinées au rasage de la peau à base de polyorganosiloxanes à fonction hydroxyalkyle et leur procédé de mise en oeuvre.

Certains polyorganosiloxanes sont bien connus dans le domaine des compositions de rasage et sont utilisés dans les formulations de crèmes à raser, de lotions avant rasage et de mousses à raser. Il s'agit principalement des silicones volatiles telles que le polydiméthylsiloxane ou "diméthicone" ou le polydiméthylcyclosiloxane ou "cyclométhicone".

On connait également des compositions de rasage à base de diméthylpolysiloxanes comprenant des fonctions hydroxyle. Elles ont été décrites dans la demande de brevet DE 1467863.

Ces silicones, utilisées dans les compositions à raser, ont pour fonction d'atténuer, par leur pouvoir lubrifiant, le "feu" du rasoir, de rendre la peau plus douce et plus satinée et de conférer aux compositions qui les referment un certain confort.

Cependant, ces silicones sont également connues pour leur propriété antimoussante et sont difficilement utilisables dans les mousses à raser conditionnées en aérosol. Lorsque ces silicones permettent la formation d'une mousse, celle-ci présente généralement une qualité et une stabilité très variables suivant le degré de remplissage du récipient conditionné en aérosol.

La demanderesse a découvert, d'une façon surprenante, que l'utilisation de polyorganosiloxanes à fonction hydroxyalkyle dans des compositions à raser, permettait d'améliorer sensiblement l'onctuosité et la douceur de ces compositions en plus des propriétés indiquées ci-dessus pour les silicones.

La demanderesse a découvert également que l'utilisation de ces polyorganosiloxanes à fonction hydroxyalkyle dans les mousses à raser permettaient à celles-ci de conserver leur qualité au cours de la vidange progressive du récipient conditionné en aérosol.

Enfin, la demanderesse a découvert que les compositions conformes à l'invention contenant des polyorganosiloxanes à fonction hydroxyalkyle, amélioraient la coupe du poil au cours du rasage, s'éliminaient très facilement au rinçage à l'eau, aussi bien de la peau que des lames de rasoir, et laissaient la peau rapidement nette et satinée.

Un objet de l'invention est constitué par les compositions destinées au rasage de la peau, contenant les polyorganosiloxanes à fonction hydroxyalkyle.

L'invention a également pour objet un procédé de rasage de la peau mettant en oeuvre ces compositions.

D'autres objets de l'invention apparaîtront à la lecture de la description et des exemples qui suivent.

La présente invention a donc pour objet une composition destinée au rasage de la peau, contenant dans un milieu cosmétiquement acceptable un agent moussant et au moins un polyorganosiloxane à fonction hydroxyalkyle, répondant à la formule (I) suivante :

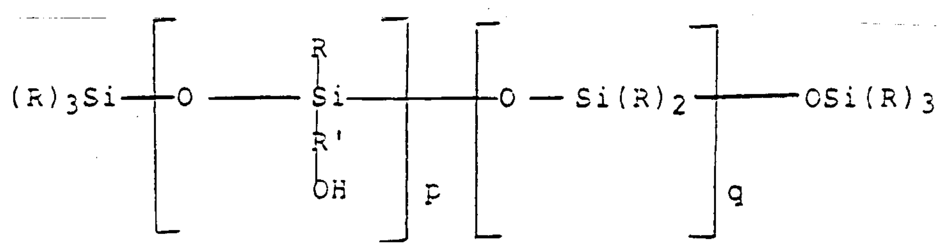

(I)

dans laquelle :

les radicaux R, identiques ou différents, sont choisis parmi les radicaux méthyle et phényle, 60% molaire au moins des radicaux R étant des radicaux méthyle;

le radical R' est un chaînon alkylène divalent hydrocarboné, linéaire ou ramifié, comportant de 2 à 18 atomes de carbone;

p est un nombre entier compris entre 1 et 30 inclus, de préférence entre 1 et 20 inclus;

q est un nombre entier compris entre 1 et 150 inclus.

Le copolymère selon l'invention peut être un copolymère alterné ou statistique.

Parmi les composés particulièrement préférés, utilisés conformément à l'invention, on peut utiliser des composés de formule (I), dans laquelle :

R' représente un chaînon alkylène divalent hydrocarboné, linéaire ou ramifié, contenant 2 à 6 atomes de carbone et plus particulièrement, une chaîne triméthylène :

$$-(CH_2)_3-$$

ou un chaînon méthyl-2 triméthylène :

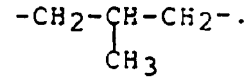

Parmi les produits de formule (I), on préfère utiliser les produits dont la masse moléculaire en nombre est comprise entre 600 et 10.000.

Des produits de formule (I) préférés sont représentés par les produits de masse moléculaire en nombre compris entre 1.000 et 10.000, pour lesquels p est compris entre 1 et 5 et q est compris entre 10 et 120.

Plus particulièrement encore, on préfère des produits de formule (I) de masse moléculaire comprise entre 8.000 et 10.000, pour lesquels p est compris entre 1 et 3, q entre 100 et 120, et parmi ceux-ci les composés de formule (I) dans lesquels R représente un radical méthyle et R′ représente un radical triméthylène $-(CH_2)_3-$.

Les produits de formule (I) sont connus en eux-mêmes et peuvent être préparés suivant les procédés connus de l'art antérieur, tels que ceux décrits dans la demande de brevet français n° 2589476, les brevets US-A-2.970.150 et 4.160.775.

Pour préparer les produits de formule (I), on peut par exemple utiliser comme organopolysiloxanes de départ, le copolymère de formule (II) :

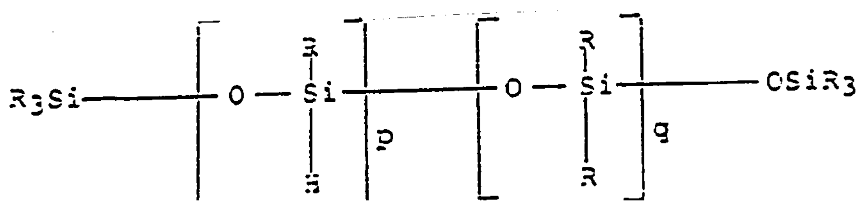
(II)

dans laquelle R, p et q ont la signification donnée ci-dessus.

Les produits de formule (II) sont bien connus dans l'industrie des silicones et sont généralement disponibles dans le commerce. Ils sont par exemple décrits dans les brevets US-A-3.220.942, 3.341.111 et 3.436.366.

Sur les produits de formule (II), on fait réagir un alcool à insaturation alcénique de formule (III) :

R″OH

dans laquelle R″ est un radical alcényle linéaire ou ramifié, ayant de 2 à 18 atomes de carbone.

Parmi ces alcools, on utilise plus particulièrement l'alcool allylique et l'alcool méthallylique.

On peut utiliser comme catalyseur d'hydrosilylation pour faire réagir les alcools insaturés de formule (III) sur l'hydrogénopolysiloxane de formule (II), des catalyseurs d'hydrosilylation connus, notamment les complexes du platine décrits dans les brevets US-A-3.715.334, 3.775.452 et 3.814.730; les complexes platine oléfine décrits dans les brevets US-A-3.159.601 et 3.159.662.

Le milieu cosmétiquement acceptable, conforme à la présente invention, est un milieu aqueux contenant au moins un agent moussant.

L'agent moussant, conforme à la présente invention, est choisi parmi les tensio-actifs anioniques synthétiques moussants ou les savons constitués d'acides gras en $C_8$-$C_{20}$, neutralisés par un agent alcalin tel que la triéthanolamine, l'hydroxyde de potassium, l'hydroxyde de sodium ou leurs mélanges.

Les tensio-actifs anioniques utilisés, conformément à la présente invention, sont choisis de préférence parmi les sels de métaux alcalins et d'ammonium des acides N-acylglutamique, alkyliséthionique, alkylsulfosuccinique, alkylsulfoacétique, N-acylsarcosine, N-acyl, N-méthyltaurine, les radicaux alkyle et acyle comportant 14 à 22 atomes de carbone.

Les acides gras utilisés conformément à la présente invention, sont de préférence constitués de mélanges d'acides gras en $C_{16}$-$C_{20}$ et d'acides gras en $C_8$-$C_{20}$, et notamment des mélanges d'acide stéarique et d'acide gras de coprah ou d'acide stéarique et d'acide myristique ou encore d'acide stéarique et d'acide laurique.

La proportion en savon d'acides gras en $C_{16}$-$C_{20}$ est comprise entre 40 et 90% et de préférence entre 75 et 90% en poids par rapport au poids total de la quantité de savon; celle en savon d'acide gras en $C_8$-$C_{20}$ de 10 à 60% et de préférence de 10 à 25% en poids par rapport au poids total de la quantité de savon.

D'autres agents moussants peuvent être utilisés en association avec les savons d'acides gras ou les tensio-actifs anioniques moussants définis ci-dessus, comme par exemple des agents tensio-actifs non-ioniques, amphotères ou anioniques, différents de ceux cités précédemment, des polymères comme l'alcool polyvinylique.

Les compositions préférées conformes à l'invention, renferment de 10 à 85% en poids d'eau, de 0,5 à 80% en poids de savon constitué d'acides gras en $C_8$-$C_{20}$ et neutralisés par un agent alcalin ou de tensio-actifs

anioniques tels que définis ci-dessus.

Les compositions de rasage de la peau conformes à l'invention, contiennent dans le milieu cosmétiquement acceptable, défini ci-dessus, un polyorganosiloxane à fonction hydroxyalkyle de formule (I) dans des concentrations comprises entre O,2 et 3% en poids et de préférence entre O,5 et 2% en poids par rapport au poids total de la composition.

Ces compositions peuvent se présenter sous forme de crèmes, de gels, de gels automoussants, de savons solides et de mousses aérosol.

Les compositions de rasage conformes à l'invention peuvent être conditionnées en aérosol pour être distribuées sous forme de mousses ou de gels automoussants.

On utilise dans ce cas la composition en présence d'un gaz propulseur choisi parmi les hydrocarbures volatils, tels que le n-butane, l'isobutane, le propane, dont on préfère plus particulièrement un mélange ternaire de n-butane, isobutane, propane, vendu par exemple par la Société ELF AQUITAINE sous la dénomination AEROGAZ 3,2 N ou des hydrocarbures partiellement ou totalement fluorés dont plus particulièrement le monofluorotrichlorométhane, le dichlorodifluorométhane (F12), le dichloro-1,2 tétrafluoro-1,1,2,2-éthane (F114), utilisés seuls ou en combinaison; des hydrocarbures chlorés et/ou fluorés de ce type sont vendus sous la dénomination de Fréon ou Dymel par la Société DU PONT DE NEMOURS. On peut également utiliser comme propulseur des mélanges de ces hydrocarbures volatils avec des hydrocarbures chlorés et/ou fluorés, comme par exemple un mélange de n-butane, isobutane, propane et de monofluorotrichlorométhane, ou des propulseurs comme le protoxyde d'azote, le gaz carbonique ou l'éther diméthylique.

Les compositions de rasage plus particulièrement préférées, selon l'invention, sont représentées par des mousses aérosol qui renferment de 75 à 85% en poids d'eau, de O,5 à 15% en poids de savon ou de tensio-actif anionique tel que décrit ci-dessus, de O,2 à 3% en poids d'un polyorganosiloxane de formule (I), dans laquelle R′ désigne

$$-(CH_2)_3-,$$

de masse moléculaire en nombre comprise entre 8.OOO et 1O.OOO pour lesquels p est compris entre 1 et 3 et q entre 1OO et 12O, et de 2 à 15% et plus particulièrement de 3 à 1O% en poids d'un agent propulseur.

Les compositions conformes à l'invention peuvent contenir en plus du polyorganosiloxane de formule (I), des adjuvants habituellement utilisés dans le domaine des compositions à raser, tels que des agents hydratants choisis parmi le sorbitol et la glycérine; des agents de transparence jouant également de rôle de solvants, tels que des glycols comme le propylèneglycol ou l'éthylèneglycol, des agents filmogènes, des agents adoucissants, comme par exemple des dérivés de lanoline ou des polymères cationiques ou des polyéthylèneglycols, des agents de traitement de la peau, tels que des agents anti-acnéiques, des antiséborrhéiques, des huiles minérales, des alcools gras, des polymères, des épaississants, des agents stabilisants, comme par exemple des éthanolamides, des agents apaisants, tels que l'allantoïne, le camphre, le menthol, des agents tensio-actifs anioniques autres que les savons ou ceux définis ci-dessus, des tensio-actifs non-ioniques, amphotères ou leurs mélanges, des colorants, des conservateurs, des antioxydants et des parfums.

Le procédé de rasage de la peau, conforme à la présente invention, consiste essentiellement à appliquer sur la peau une composition telle que définie ci-dessus, à raser celle-ci au moyen d'un rasoir mécanique et à faire suivre d'un rinçage à l'eau.

Les exemples suivants sont destinés à mieux illustrer l'invention.

EXEMPLE 1

On prépare une mousse aérosol de rasage de composition suivante :

- Acide stéarique 8,0 g
- Acide gras de coprah 1,0 g
- Hydroxyde de potassium 0,5 g
- Triéthanolamine 3,0 g
- Polysiloxane de formule :

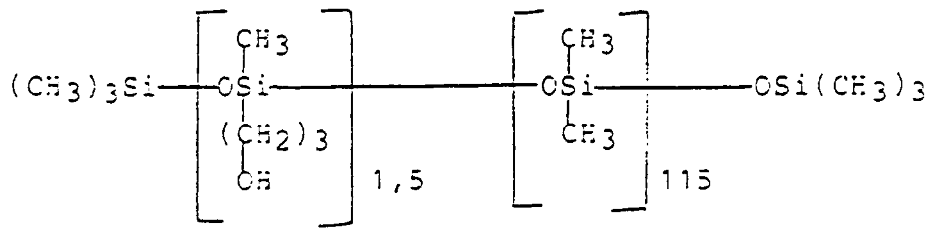

(Mn ≃ 9.000) 1,0 g

- Glycérine 4,0 g
- Sels d'isopropanolamines de l'acide myristique, vendus sous la dénomination LANAMINE par la Société AMERCHOL 2,0 g
- Monolaurate de sorbitan polyoxyéthyléné à 20 moles d'oxyde d'éthylène, vendu sous la dénomination TWEEN 20 par la Société ATLAS 0,5 g
- Parfum qs
- Eau qsp 100,0 g

Conditionnement aérosol :

Composition ci-dessus 96,0 g

Propulseur :
Mélange ternaire de n-butane isobutane > 55%, propane vendu sous la dénomination AEROGAZ 3,2 N par la Société ELF AQUITAINE 4,0 g

Total 100,0 g

EXEMPLE 2

On prépare une mousse aérosol de rasage de composition suivante :

| | |
|---|---|
| - Acide stéarique | 5,5 g |
| - Acide myristique | 1,2 g |
| - Hydroxyde de potassium | 0,4 g |
| - Triéthanolamine | 3,3 g |
| - Glycérine | 2,5 g |
| - Polysiloxane de formule : | |

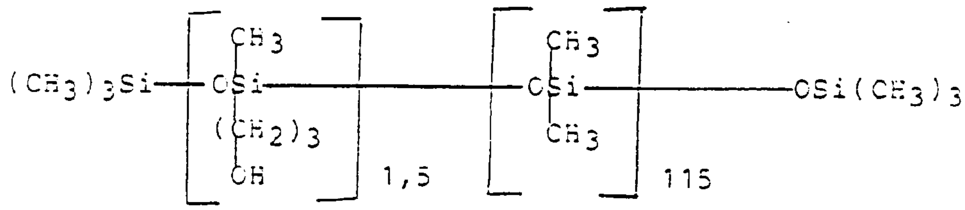

| | |
|---|---|
| (Mn ≃ 9.000) | 1,0 g |
| - Acide lanolique | 0,5 g |
| - Tensio-actif amphotère dénommé "Cocoamphocarboxyglycinate" (CTFA, 3ème édition, 1982), vendu sous le nom de MIRANOL C2M Conc, par la Société MIRANOL, en solution aqueuse à 38% de matière active | 0,2 g MA |

| | | | | |
|---|---|---|---|---|
| - Copolymère de diméthyldiallyl ammonium et d'acrylamide, vendu en solution aqueuse à 8% de matière active (MA) sous la dénomination MERQUAT S par la Société MERCK | | | 0,25 | g MA |
| - Parfum | qs | | | |
| - Eau | | qsp | 100,0 | g |

Conditionnement aérosol :

| | | | |
|---|---|---|---|
| Composition ci-dessus | | 92,0 | g |
| Propulseur : Fréons 12/114 (53/47) | | 8,0 | g |
| | Total | 100,0 | g |

EXEMPLE 3

On prépare un gel de rasage auto-moussant de composition suivante :

| | |
|---|---|
| - Acide stéarique | 5,0 g |
| - Acide palmitique | 5,0 g |
| - Triéthanolamine | 5,5 g |
| - Polysiloxane de formule : | |

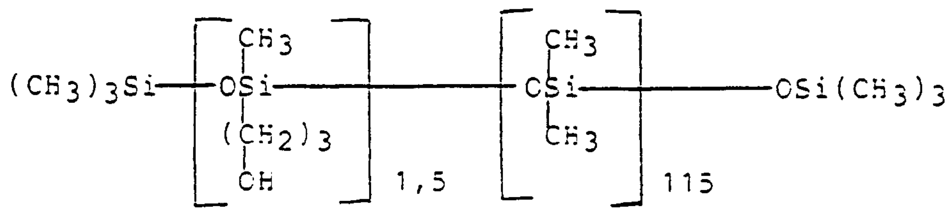

| | |
|---|---|
| (Mn ∼ 9.000) | 0,5 g |
| - Polyéthylèneglycol vendu sous la dénomination POLYOX WSR 205 par la Société UNION CARBIDE | 0,55 g |
| - Polyéthylèneglycoléther (20 OE) d'alcool oléique, vendu sous la dénomination BRIJ 98 par la Société ICI AMERICAS | 2,0 g |
| - Hydroxyéthylcellulose vendue par la Société UNION CARBIDE sous la dénomination CELLOSIZE PCG 10 | 1,4 g |
| - Sorbitol | 1,0 g |
| - Parfum, colorant | qs |
| - Eau | 100,0 g |

On introduit 96 g de cette composition dans la partie centrale d'un aérosol à double enveloppe, dont la paroi interne est constituée d'une membrane compressible, imperméable, séparant le propulseur (enveloppe extérieure) de la composition gélifiée auto-moussante (partie centrale).

On introduit ensuite 4 g d'un mélange isopentane (60) Fréon 114 (40) dans la partie centrale. Après sertissage de la valve, on pressurise la bombe aérosol en introduisant dans la double enveloppe 10% d'un mélange ternaire de n-butane, isobutane > 55%, propane vendu sous la dénomination AEROGAZ 3,2 N par la Société ELF AQUITAINE.

Appliqué sur la peau, ce gel développe une mousse très rapidement.

EXEMPLE 4

On prépare une crème à raser de composition suivante :

| | | |
|---|---|---|
| - Acide stéarique | | 34,0 g |
| - Acide gras de coprah | | 8,5 g |
| - Hydroxyde de potassium | | 7,5 g |
| - Hydroxyde de sodium | | 0,75 g |
| - Glycérine | | 13,5 g |
| - Polysiloxane de formule : | | |

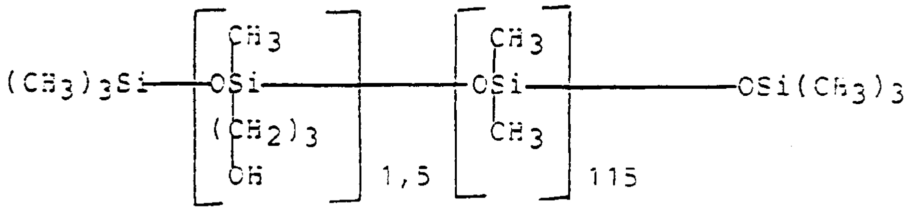

| | | |
|---|---|---|
| (Mn ≃ 9.000) | | 2,0 g |
| - Antioxydant, parfum | qs | |
| - Eau | qsp | 100,0 g |

EXEMPLE 5

On prépare une mousse à raser de composition suivante :

| | |
|---|---|
| - N-méthyloléyltaurate de sodium, vendu par la Société GAF sous la dénomination FENOPON TK 42 à la concentration de 32% MA (matière active) | 4,8 g MA |
| - Lauryléther sulfate de sodium à 70% MA | 2,1 g MA |
| - Sorbitol à 70% MA | 5,6 g MA |
| - Acide lanolique | 0,5 g |
| - Monolaurate de sorbitan, polyoxyéthyléné à 20 moles d'oxyde d'éthylène, vendu par la Société ATLAS sous la dénomination TWEEN 20 | 0,5 g |
| - Copolymère de chlorure de diméthyldiallylammonium et d'acrylamide, vendu en solution aqueuse à 8% MA par la Société GAF sous la dénomination MERQUAT S | 0,08 g MA |
| - Polysiloxane de formule : | |

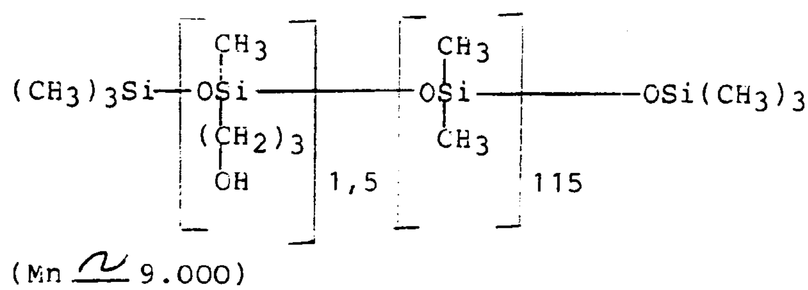

| | |
|---|---|
| (Mn ~ 9.000) | 2,0 g |
| - Hydroxyéthylcellulose vendue par la Société UNION CARBIDE sous la dénomination CELLOSIZE PCG 10 | 1,5 g |

| | | |
|---|---|---|
| - D-panthénol en solution à 50% MA | | 0,375 g MA |
| - Colorant, parfum | qs | |
| - Eau | qsp | 100,0 g |

<u>Conditionnement aérosol</u> :

| | | | |
|---|---|---|---|
| Composition ci-dessus | | 94,0 | g |
| Propulseur : Isobutane | | 6,0 | g |
| | TOTAL | 100,0 | g |

EXEMPLE 6

On prépare une mousse à raser de composition suivante :

| | |
|---|---|
| - Sel monosodique de N-acylglutamate de formule :<br>HOOC - $CH_2$ - $CH_2$ - CH - COONa<br>NH - COR<br>où R est un mélange de radicaux alkényle et/ou alkyle hydrogénés en $C_{14}$-$C_{22}$ dérivés des acides gras du suif, vendu sous la dénomination ACYLGLUTAMATE HS 11 par la Société AJINOMOTO | 4,0 g |
| - Sel dissodique de N-acylglutamate de formule ci-dessus, vendu sous la dénomination ACYLGLUTAMATE HS 21 par la Société AJINOMOTO | 3,0 g |
| - Glycérine | 3,0 g |
| - Acide lanolique | 1,0 g |
| - Tensio-actif amphotère dénommé "Cocoamphocarboxyglycinate" (CTFA, 3ème édition, 1982), vendu sous la dénomination MIRANOL C2M Conc., par la Société MIRANOL, en solution aqueuse à 38% de matière active (MA) | 0,38 g MA |
| - Hydroxyéthylcellulose vendue par la Société UNION CARBIDE sous la dénomination CELLOSIZE PCG 10 | 1,0 g |
| - Polyéthylèneglycol vendu sous la dénomination POLYOX WSR 205 par la Société UNION CARBIDE | 0,5 g |

- Polyéthylèneglycol vendu sous la dénomination POLYOX COAGULANT par la Société UNION CARBIDE — 0,05 g
- Polysiloxane de formule :

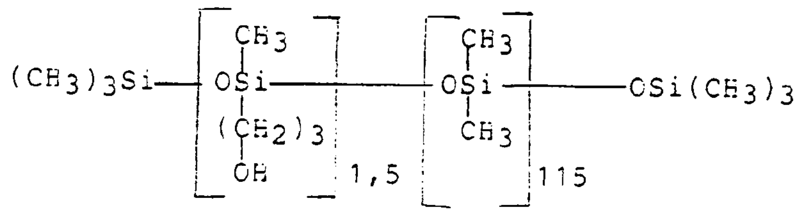

(Mn ≃ 9.000) — 1,0 g

- Parfum — qs
- Eau — qsp 100,0 g

<u>Conditionnement aérosol</u> :

| | |
|---|---|
| Composition ci-dessus | 96,0 g |
| Propulseur : Mélange ternaire de n-butane isobutane > 55%, propane vendu sous la dénomination AEROGAZ 3,2 N par la Société ELF AQUITAINE | 4,0 g |
| TOTAL | 100,0 g |

## Revendications

### Revendications pour les Etats contractants suivants : AT BE CH DE FR GB GR IT LI NL SE

1. Composition moussante pour le rasage de la peau, caractérisée par le fait qu'elle contient dans un milieu aqueux, au moins
un polyorganosiloxane à fonction hydroxyalkyle, répondant à la formule (I) suivante :

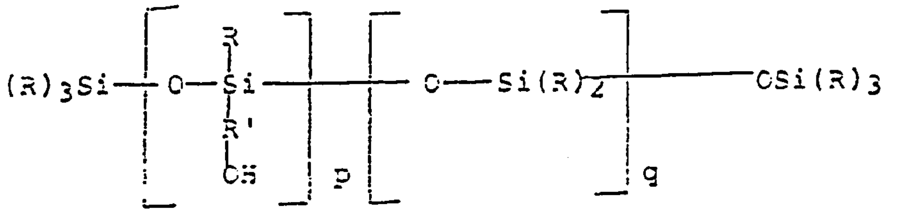 (I)

dans laquelle les radicaux R, identiques ou différents, sont choisis parmi les radicaux méthyle et phényle, 60% molaire au moins des radicaux R étant des radicaux méthyle, le radical R′ est un chaînon alkylène divalent hydrocarboné, linéaire ou ramifié, comportant de 2 à 18 atomes de carbone; p est un nombre entier compris entre 1 et 30 inclus; q est un nombre entier compris entre 1 et 150 inclus et un agent moussant.

2. Composition selon la revendication 1, caractérisée par le fait que dans les composés de formule (I), R′ représente un chaînon alkylène divalent hydrocarboné, linéaire ou ramifié, comportant de 2 à 6 atomes de carbone.

3. Composition selon la revendication 1 ou 2, caractérisée par le fait que dans les composés de formule (I), R′ est le radical triméthylène :

$$-(CH_2)_3-$$

ou un chaînon méthyl-2 triméthylène :

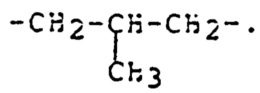

4. Composition selon l'une des revendications 1 à 3, caractérisée par le fait que dans les composés de formule (I), R représente un radical méthyle, R′ un radical triméthylène, p est un nombre entier compris entre 1 et 3 et q est un nombre entier compris entre 100 et 120, ces composés ayant une masse moléculaire en nombre comprise entre 8.000 et 10.000.

5. Composition selon l'une quelconque des revendications 1 à 4, caractérisée par le fait qu'elle contient le polyorganosiloxane de formule (I), dans des concentrations comprises entre 0,2 et 3% en poids et de préférence entre 0,5 et 2% en poids par rapport au poids total de la composition.

6. Composition selon l'une quelconque des revendications 1 à 5, caractérisée par le fait qu'elle se présente sous forme de crème, de gel, de gel automoussant, de savon solide ou de mousse aérosol.

7. Composition selon l'une quelconque des revendications 1 à 6, caractérisée par le fait qu'elle contient un agent moussant constitué par un acide gras en $C_8$-$C_{20}$ neutralisé par un agent alcalin ou un tensio-actif anionique synthétique.

8. Composition selon la revendication 7, caractérisée par le fait que l'agent tensio-actif anionique est choisi parmi les sels de métaux alcalins et d'amonium des acides N-acylglutamique, alkyliséthionique, alkylsulfosuccinique, alkylsulfoacétique, N-acylsarcosine, N-acyl N-méthyltaurine, les radicaux alkyle et acyle comportant de 14 à 22 atomes de carbone.

9. Composition selon la revendication 7, caractérisée par le fait que l'agent moussant est un savon constitué d'un mélange d'acides gras en $C_8$-$C_{20}$ et d'acides gras en $C_{16}$-$C_{20}$, que la proportion en acides gras en $C_8$-$C_{20}$ est comprise entre 10 et 60% et de préférence entre 10 et 25% en poids par rapport au poids total de la quantité de savon et que la proportion en d'acides gras en $C_{16}$-$C_{20}$, que la proportion en acides gras en $C_8$-$C_{20}$ est compris entre 10 et 60% et de préférence entre 10 et 25% en poids par rapport au poids total de la quantité de savon et que la proportion en acides gras en $C_{16}$-$C_{20}$ est comprise entre 40 et 90% et de préférence entre 75% et 90% en poids par rapport au poids total de la quantité de savon.

10. Composition selon l'une quelconque des revendications 5 à 9, caractérisée par le fait qu'elle renferme, de 10 à 85% en poids d'eau et de 0,5 à 80% en poids d'agent moussant, tel que défini dans les revendications 7 à 9.

11. Composition selon l'une quelconque des revendications 5 à 10, caractérisée par le fait qu'elle contient en plus un polymère moussant ou un agent tensio-actif moussant non-ionique, amphotère ou anionique différent de ceux définis dans la revendication 8.

12. Composition selon l'une quelconque des revendications 5 à 11, caractérisée par le fait qu'elle est conditionnée en aérosol en présence d'un gaz propulseur pour former au moment de l'expulsion, un gel auto-moussant ou une mousse.

13. Composition selon la revendication 12, caractérisée par le fait qu'elle se présente sous forme de mousse aérosol, qu'elle referme 75 à 85% en poids d'eau, O,5 à 15% en poids d'agent moussant tel que défini dans les revendications 7 à 9, O,2 à 3% en poids d'un organopolysiloxane de formule (I) et de 2 à 15% en poids d'un gaz propulseur et de préférence 3 à 1O% en poids par rapport au poids total de la composition.

14. Composition selon l'une quelconque des revendications 5 à 13, caractérisée par le fait qu'elle contient en plus des agents hydratants, des agents de transparence, des agents filmogènes, des agents adoucissants, des agents anti-acnéiques, des anti-séborrhéiques, des huiles minérales, des alcools gras, des polymères, des épaississants, des agents stabilisants, des agents apaisants, des agents tensio-actifs anioniques autres que des savons ou ceux définis dans la revendication 8, des tensio-actifs non-ioniques, amphotères ou leurs mélanges, des colorants, des conservateurs, des antioxydants et des parfums.

15. Procédé de rasage de la peau, caractérisé par le fait que l'on applique sur la peau une composition telle que définie dans l'une quelconque des revendications 1 à 14, que l'on rase la peau au moyen d'un rasoir mécanique et que l'on rince à l'eau.

**Revendications pour l'Etat contractant suivant : ES**

1. Procédé de préparation d'une composition moussante pour le rasage de la peau, caractérisé par le fait que l'on incorpore dans un milieu aqueux, au moins un polyorganosilixane à fonction hydroxyalkyle, répondant à la formule (I) suivante :

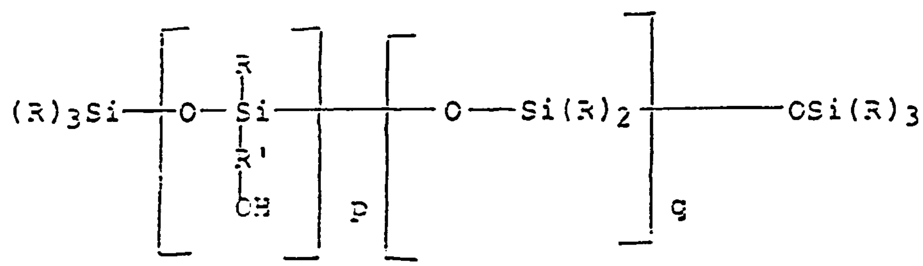

(I)

dans laquelle les radicaux R, identiques ou différents sont choisis parmi les radicaux méthyle et phényle, 6O% molaire au moins des radicaux R étant des radicaux méthyle, le radical R' est un chaînon alkylène divalent hydrocarboné, linéaire ou ramifié, comportant de 2 à 16 atomes de carbon; p est un nombre entier compris entre 1 et 3O inclus; q est un nombre entier compris entre 1 et 15O inclus et un agent moussant.

2. Procédé selon la revendication 1, caractérisée par le fait que dans les composés de formule (I), R' représente un chaînon alkylène divalent hydrocarboné, linéaire ou ramifié, comportant de 2 à 6 atomes de carbone.

3. Procédé selon la revendication 1 ou 2, caractérisée par le fait que dans les composés de formule (I), R' est le radical triméthylène :

$$-(Ch_2)_3-$$

ou un chaînon méthyl-2 triméthylène :

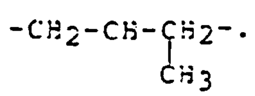

4. Procédé selon l'une des revendications 1 à 3, caractérisée par le fait que dans les composés de formule (I), R représente un radical méthyle, R' un radical triméthylène, p est un nombre entier compris entre 1 et 3 et q est un nombre entier compris entre 1OO et 12O, ces composés ayant une masse moléculaire en nombre comprise entre 8.OOO et 1O.OOO.

5. Composition moussante pour le rasage de la peau, caractérisée par le fait qu'elle contient dans un milieu aqueux, au moins un polyorganosiloxane de formule (I) tel que défini dans les revendications 1 à 4 et un agent moussant.

6. Composition selon la revendication 5, caractérisée par le fait qu'elle se présente sous forme de crème, de gel, de gel auto-moussant, de savon solide ou de mousse aérosol.

7. Composition selon l'une des revendications 5 et 6, caractérisée par le fait qu'elle contient un agent moussant constitué par un acide gras en $C_8$-$C_{20}$ neutralisé par un agent alcalin ou un tensio-actif anionique synthétique.

8. Composition selon la revendication 7, caractérisée par le fait que l'agent tensio-actif anionique est choisi parmi les sels de métaux alcalins et d'ammonium des acides N-acylglutamique, alkyliséthionique, alkylsulfosuccinique, alkylsulfoacétique, N-acylsarcosien, N-acyl N-méthyltaurine, les radicaux alkyle et acyle comportant de 14 à 22 atomes de carbone.

9. Composition selon la revendication 7, caractérisée par le fait que l'agent moussant est un savon constitué d'un mélange d'acides gras en $C_8$-$C_{20}$ et d'acides gras en $C_{16}$-$C_{20}$, que la proportion en acides gras en $C_8$-$C_{20}$ est comprise entre 1O et 6O% et de préférence entre 1O et 25% en poids par rapport au poids total de la quantité de savon et que la proportion en acides gras en $C_{16}$-$C_{20}$ est comprise entre 4O et 9O% et de préférence entre 75% et 9O% en poids par rapport au poids total de la quantité de savon.

1O. Composition selon l'une quelconque des revendications 5 à 9, caractérisée par le fait qu'elle renferme, de 1O à 85% en poids d'eau et de O,5 à 8O% en poids d'agent moussant, tel que défini dans les revendications 7 à 9.

11. Composition selon l'une quelconque des revendications 5 à 10, caractérisée par le fait qu'elle contient en plus un polymère moussant ou un agent tensio-actif moussant non-ionique, amphotère ou anionique différent de ceux définis dans la revendication 8.

12. Composition selon l'une quelconque des revendications 5 à 11, caractérisée par le fait qu'elle est conditionnée en aérosol en présence d'un gaz propulseur pour former au moment de l'expulsion, un gel auto-moussant ou une mousse.

13. Composition selon la revendication 12, caractérisée par le fait qu'elle se présente sous forme de mousse aérosol, qu'elle referme 75 à 85% en poids d'eau, O,5 à 15% en poids d'agent moussant tel que défini dans les revendications 7 à 9, O,2 à 3% en poids d'un organopolysiloxane de formule (I) et de 2 à 15% en poids d'un gaz propulseur et de préférence 3 à 1O% en poids par rapport au poids total de la composition.

14. Composition selon l'une quelconque des revendications 5 à 13, caractérisée par le fait qu'elle contient en plus des agents hydratants, des agents de transparence, des agents filmogènes, des agents adoucissants, des agents anti-acnéiques, des anti-séborrhéiques, des huiles minérales, des alcools gras, des polymères, des épaississants, des agents stabilisants, des agents apaisants, des agents tensio-actifs anioniques autres que des savons ou ceux définis dans la revendication 8, des tensio-actifs non-ioniques, amphotères ou leurs mélanges, des colorants, des conservateurs, des antioxydants et des parfums.

15. Procédé de rasage de la peau, caractérisé par le fait que l'on applique sur la peau une composition telle que définie dans l'une quelconque des revendications 5 à 14, que l'on rase la peau au moyen d'un rasoir mécanique et que l'on rince à l'eau.

## Claims

### Claims for the following Contracting States : AT, BE, CH, DE, FR, GB, GR, IT, LI, NL, SE

1. Foaming composition for shaving the skin, characterised in that it contains, in an aqueous medium, at least one polyorganosiloxane containing a hydroxyalkyl functional group, corresponding to the following formula (I):

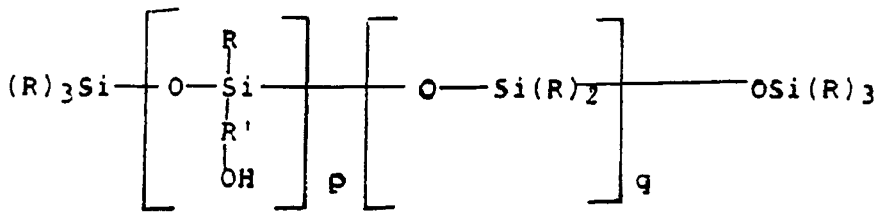

(I)

in which the radicals R, which are identical or different, are chosen from methyl and phenyl radicals, at least 60 mol% of the radicals R being methyl radicals, the radical R' is a linear or branched hydrocarbon divalent alkylene chain unit containing from 2 to 18 carbon atoms, p is an integer between 1 and 30 inclusive, q is an integer between 1 and 150 inclusive, and a foaming agent.

**2.** Composition according to Claim 1, characterised in that, in the compounds of formula (I), R′ denotes a linear or branched hydrocarbon divalent alkylene chain unit containing from 2 to 6 carbon atoms.

**3.** Composition according to Claim 1 or 2, characterised in that, in the compounds of formula (I), R′ is the trimethylene radical: $-(CH_2)_3-$ or a 2-methyltrimethylene chain unit:

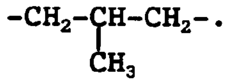

**4.** Composition according to one of Claims 1 to 3, characterised in that, in the compounds of formula (I), R denotes a methyl radical, R′ a trimethylene radical, p is an integer between 1 and 3 and q is an integer between 100 and 120, these compounds having a number molecular mass of between 8,000 and 10,000.

**5.** Composition according to any one of Claims 1 to 4, characterised in that it contains the polyorganosiloxane of formula (I) in concentrations of between 0.2 and 3% by weight and preferably between 0.5 and 2% by weight relative to the total weight of the composition.

**6.** Composition according to any one of Claims 1 to 5, characterised in that it is in the form of cream, gel, self-foaming gel, solid soap or aerosol foam.

**7.** Composition according to any one of Claims 1 to 6, characterised in that it contains a foaming agent consisting of a $C_8$-$C_{20}$ fatty acid neutralised by an alkaline agent or a synthetic anionic surfactant.

**8.** Composition according to Claim 7, characterised in that the anionic surface-active agent is chosen from alkali metal and ammonium salts of N-acylglutamic, alkylisethionic, alkylsulphosuccinic and alkylsulphoacetic acids, N-acylsarcosine and N-acyl-N-methyltaurine, the alkyl and acyl radicals containing from 14 to 22 carbon atoms.

**9.** Composition according to Claim 7, characterised in that the foaming agent is a soap consisting of a mixture of $C_8$-$C_{20}$ fatty acids and $C_{16}$-$C_{20}$ fatty acids, that the proportion of $C_8$-$C_{20}$ fatty acids is between 10 and 60% and preferably between 10 and 25% by weight relative to the total weight of the quantity of soap and that the proportion of $C_{16}$-$C_{20}$ fatty acids is between 40 and 90% and preferably between 75% and 90% by weight relative to the total weight of the quantity of soap.

**10.** Composition according to any one of Claims 5 to 9, characterised in that it contains from 10 to 85% by weight of water and from 0.5 to 80% by weight of foaming agent as defined in Claims 7 to 9.

**11.** Composition according to any one of Claims 5 to 10, characterised in that it additionally contains a foaming polymer or a nonionic, amphoteric or anionic foaming surface-active agent other than those defined in Claim 8.

**12.** Composition according to any one of Claims 5 to 11, characterised in that it is packaged as an aerosol in the presence of a propellent gas to form a self-foaming gel or a foam at the time of the expulsion.

**13.** Composition according to Claim 12, characterised in that it is in the form of aerosol foam, that it contains 75 to 85% by weight of water, 0.5 to 15% by weight of foaming agent as defined in Claims 7 to 9, 0.2 to 3% by weight of an organopolysiloxane of formula (I) and from 2 to 15% by weight of a propellent gas and preferably 3 to 10% by weight relative to the total weight of the composition.

**14.** Composition according to any one of Claims 5 to 13, characterised in that it additionally contains hydrating agents, transparency agents, film-forming agents, emollient agents, antiacne agents, antiseborrhoeic agents, mineral oils, fatty alcohols, polymers, thickeners, stabilising agents, soothing agents, anionic surface-active agents other than soaps or those defined in Claim 8, nonionic or amphoteric surfactants or mixtures thereof, colorants, preserving agents, antioxidants and perfumes.

**15.** Process for shaving the skin, characterised in that a composition as defined in any one of Claims 1 to 14 is applied to the skin, that the skin is shaved with a mechanical razor and that rinsing with water is carried out.

**Claims for the following Contracting State : ES**

1. Process for the preparation of a foaming composition for shaving the skin, characterised in that at least one polyorganosiloxane containing a hydroxyalkyl functional group, corresponding to the following formula

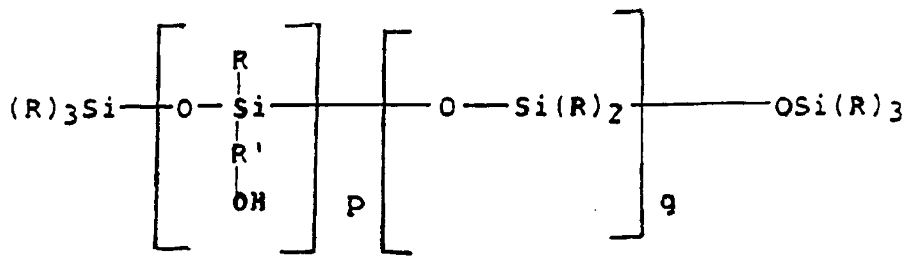

(I)

in which the radicals R, which are identical or different, are chosen from methyl and phenyl radicals, at least 60 mol% of the radicals R being methyl radicals, the radical R′ is a linear or branched hydrocarbon divalent alkylene chain unit containing from 2 to 18 carbon atoms, p is an integer between 1 and 30 inclusive and q is an integer between 1 and 150 inclusive, and a foaming agent are incorporated into an aqueous medium.

2. Process according to Claim 1, characterised in that, in the compounds of formula (I), R′ denotes a linear or branched hydrocarbon divalent alkylene chain unit containing from 2 to 6 carbon atoms.

3. Process according to Claim 1 or 2, characterised in that, in the compounds of formula (I), R′ is the trimethylene radical: $-(CH_2)_3-$ or a 2-methyltrimethylene chain unit:

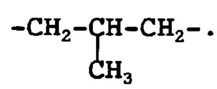

4. Process according to one of Claims 1 to 3, characterised in that, in the compounds of formula (I), R denotes a methyl radical, R′ a trimethylene radical, p is an integer between 1 and 3 and q is an integer between 100 and 120, these compounds having a number molecular mass of between 8,000 and 10,000.

5. Foaming composition for shaving the skin, characterised in that it contains, in an aqueous medium, at least one polyorganosiloxane of formula (I) as defined in Claims 1 to 4 and a foaming agent.

6. Composition according to Claim 5, characterised in that it is in the form of cream, gel, self-foaming gel, solid soap or aerosol foam.

7. Composition according to either of Claims 5 and 6, characterised in that it contains a foaming agent consisting of a $C_8$-$C_{20}$ fatty acid neutralised by an alkaline agent or a synthetic anionic surfactant.

8. Composition according to Claim 7, characterised in that the anionic surface-active agent is chosen from alkali metal and ammonium salts of N-acylglutamic, alkylisethionic, alkylsulphosuccinic and alkylsulphoacetic acids, N-acylsarcosine and N-acyl-N-methyltaurine, the alkyl and acyl radicals containing from 14 to 22 carbon atoms.

9. Composition according to Claim 7, characterised in that the foaming agent is a soap consisting of a mixture of $C_8$-$C_{20}$ fatty acids and of $C_{16}$-$C_{20}$ fatty acids, that the proportion of $C_8$-$C_{20}$ fatty acids is between 10 and 60% and preferably between 10 and 25% by weight relative to the total weight of the quantity of soap and that the proportion of $C_{16}$-$C_{20}$ fatty acids is between 40 and 90% and preferably between 75% and 90% by weight relative to the total weight of the quantity of soap.

10. Composition according to any one of Claims 5 to 9, characterised in that it contains from 10 to 85% by weight of water and from 0.5 to 80% by weight of foaming agent as defined in Claims 7 to 9.

**11.** Composition according to any one of Claims 5 to 10, characterised in that it additionally contains a foaming polymer or a nonionic, amphoteric or anionic foaming surface-active agent other than those defined in Claim 8.

**12.** Composition according to any one of Claims 5 to 11, characterised in that it is packaged as an aerosol in the presence of a propellent gas to form a self-foaming gel or a foam at the time of the expulsion.

**13.** Composition according to Claim 12, characterised in that it is in the form of aerosol foam, that it contains 75 to 85% by weight of water, 0.5 to 15% by weight of foaming agent as defined in Claims 7 to 9, 0.2 to 3% by weight of an organopolysiloxane of formula (I) and from 2 to 15% by weight of a propellent gas and preferably 3 to 10% by weight relative to the total weight of the composition.

**14.** Composition according to any one of Claims 5 to 13, characterised in that it additionally contains hydrating agents, transparency agents, film-forming agents, emollient agents, antiacne agents, antiseborrhoeic agents, mineral oils, fatty alcohols, polymers, thickeners, stabilising agents, soothing agents, anionic surface-active agents other than soaps or those defined in Claim 8, nonionic or amphoteric surfactants or mixtures thereof, colorants, preserving agents, antioxidants and perfumes.

**15.** Process for shaving the skin, characterised in that a composition as defined in any one of Claims 5 to 14 is applied to the skin, that the skin is shaved with a mechanical razor and that rinsing with water is carried out.

**Patentansprüche**

**Patentansprüche für folgende Vertragstaaten : AT, BE, CH, DE, FR, GB, GR, IT, LI, NL, SE**

I. Schäumende Zusammensetzung zum Rasieren der Haut, dadurch **gekennzeichnet**, daß sie in einem wässrigen Milieu mindestens ein Polyorganosiloxan mit Hydroxyalkylfunktion der folgenden Formel (I):

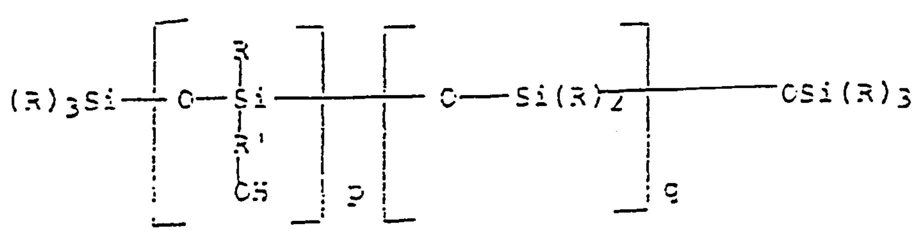

(I)

worin die Reste R, gleich oder verschieden, aus Methyl- und Phenylresten ausgewählt sind, wobei mindestens 60 Mol% der Reste R Methylreste sind, und worin der Rest R′ eine lineare oder verzweigte zweiwertige Alkylenkohlenwasserstoffkette mit 2 bis I8 Kohlenstoffatomen ist, p eine ganze Zahl von I bis 30 und q eine ganze Zahl von I bis I50 sind, sowie ein schäumendes Mittel enthält.

2. Zusammensetzung gemäß Anspruch I, dadurch **gekennzeichnet**, daß in den Verbindungen der Formel (I) R′ eine lineare oder verzweigte zweiwertige Alkylenkohlenwasserstoffkette mit 2 bis 6 Kohlenstoffatomen darstellt.

3. Zusammensetzung gemäß Anspruch I oder 2, dadurch **gekennzeichnet**, daß in den Verbindungen der Formel (I) R′ der Trimethylenrest: $-(CH_2)_3-$ oder eine Methyl-2-trimethylenkette

$$-CH_2-CH-CH_2-$$
$$CH_3$$

ist.

4. Zusammensetzung gemäß einem der Ansprüche I bis 3, dadurch **gekennzeichnet**, daß in den Verbindungen der Formel (I) R einen Methylrest, R′ einen Trimethylenrest, p eine ganze Zahl von I bis 3 und q eine ganze Zahl von I00 bis I20 darstellen, wobei diese Verbindungen eine Molmassenzahl von 8000 bis I0000 aufweisen.

5. Zusammensetzung gemäß jedem der Ansprüche I bis 4, dadurch **gekennzeichnet**, daß sie das Polyorganosiloxan der Formel (I) in Konzentrationen von 0,2 bis 3 Gew.%, vorzugsweise von 0,5 bis 2 Gew.%, bezogen auf Gesamtgewicht der Zusammensetzung, enthält.

6. Zusammensetzung gemäß jedem der Ansprüche I bis 5, dadurch **gekennzeichnet**, daß sie in der Form einer Creme, eines Gels, selbstschäumenden Gels, einer festen Seife oder eines Aerosolschaumes vorliegt.

7. Zusammensetzung gemäß jedem der Ansprüche I bis 6, dadurch **gekennzeichnet**, daß sie ein schäumendes Mittel aus einer $C_8$-$C_{20}$-Fettsäure, die mit einem alkalischen Mittel neutralisiert ist, oder ein anionisches synthetisches oberflächenaktives Mittel enthält.

8. Zusammensetzung gemäß Anspruch 7, dadurch **gekennzeichnet**, daß das anionische oberflächenaktive Mittel ausgewählt ist aus Alkalimetall- und Ammoniumsalzen von N-Acylglutamin-, Alkylthio-, Alkylsulfobernstein-, Alkylsulfoessigsäuren, N-Acylsarcosin, N-Acyl-N-methyltaurin, wobei die Alkyl- und Acylreste I4 bis 22 Kohlenstoffatome aufweisen.

9. Zusammenetzung gemäß Anspruch 7, dadurch **gekennzeichnet**, daß das schäumende Mittel eine Seife aus einer Mischung von $C_8$-$C_{20}$-Fettsäuren und $C_{I6}$-$C_{20}$-Fettsäuren ist und die Mengenverhältnisse der $C_8$-$C_{20}$-Fettsäuren I0 bis 60 Gew.%, vorzugsweise I0 bis 25 Gew.%, bezogen auf Gesamtgewicht der Seifenmenge, und die Mengenverhältnisse der $C_{I6}$-$C_{20}$-Fettsäuren 40 bis 90 Gew.%, vorzugsweise 75 bis 90 Gew.%, bezogen auf Gesamtgewicht der Seifenmenge, betragen.

I0. Zusammensetzung gemäß jedem der Ansprüche 5 bis 9, dadurch **gekennzeichnet**, daß sie I0 bis 85 Gew.% Wasser und 0,5 bis 80 Gew.% eines schäumenden Mittels gemäß der Ansprüche 7 bis 9 enthält.

II. Zusammensetzung gemäß jedem der Ansprüche 5 bis I0, dadurch **gekennzeichnet**, daß sie zusätzlich ein schäumendes Polymer oder ein schäumendes nicht-ionisches, amphoteres oder ein von den in Anspruch 8 definierten unterschiedenes anionisches oberflächenaktives Mittel enthält.

I2. Zusammensetzung gemäß jedem der Ansprüche 5 bis II, dadurch **gekennzeichnet**, daß sie als Aerosol in der Gegenwart eines Treibmittelgases zubereitet ist, um im Augenblick der Freisetzung ein selbstschäumendes Gel oder einen Schaum zu bilden.

I3. Zusammensetzung gemäß Anspruch I2, dadurch **gekennzeichnet**, daß sie in Form eines Aerosolschaumes vorliegt und 75 bis 85 Gew.% Wasser, 0,5 bis I5 Gew.% eines schäumenden Mittels gemäß der Ansprüche 7 bis 9, 0,2 bis 3 Gew.% eines Organopolysiloxans der Formel (I) und 2 bis I5 Gew.%, vorzugsweise 3 bis I0 Gew.%, eines Treibmittelgases, bezogen auf Gesamtgewicht der Zusammensetzung, enthält.

I4. Zusammensetzung gemäß jedem der Ansprüche 5 bis I3, dadurch **gekennzeichnet**, daß sie zusätzlich Hydratisierungsmittel, Transparenzmittel, filmbildende Mittel, weichmachende Mittel, Anti-Akne-Mittel, antiseborrheische Mittel, Mineralöle, Fettalkohle, Polymere, Verdickungsmittel, Stabilisierungsmittel, Beruhigungsmittel, von den Seifen oder den in Anspruch 8 definierten unterschiedene anionische oberflächenaktive Mittel, nicht-ionische, amphotere oberflächenaktive Mittel oder deren Mischungen, Färbemittel, Konservierungsmittel, Antioxidantien und Pafüms enthält.

I5. Verfahren zum Rasieren der Haut, dadurch **gekennzeichnet**, daß man auf die Haut eine Zusammensetzung gemäß jedem der Ansprüche I bis I4 aufbringt, die Haut mit einem mechanischen Rasierer rasiert und mit Wasser spült.

**Patentansprüche für folgenden Vertragstaat : ES**

I. Verfahren zur Herstellung einer schäumenden Zusammensetzung zum Rasieren der Haut, dadurch **gekennzeichnet**, daß sie in einem wässrigen Milieu mindestens ein Polyorganosiloxan mit Hydroxyalkylfunktion der folgenden Formel (I):

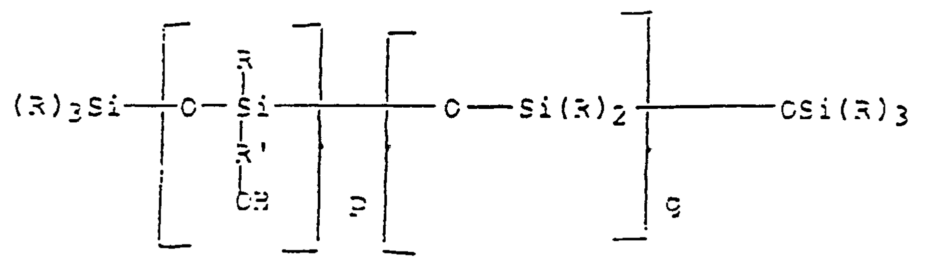 (I)

worin die Reste R, gleich oder verschieden, aus Methyl- und Phenylresten ausgewählt sind, wobei mindestens 60 Mol% der Reste R Methylreste sind, und worin der Rest R′ eine lineare oder verzweigte zweiwertige Alkylenkohlenwasserstoffkette mit 2 bis I8 Kohlenstoffatomen ist, p eine ganze Zahl von I bis 30 und q eine ganze

Zahl von I bis I50 sind, sowie ein schäumendes Mittel einbringt.

2. Verfahren gemäß Anspruch I, dadurch **gekennzeichnet**, daß in den Verbindungen der Formel (I) R′ eine lineare oder verzweigte zweiwertige Alkylenkohlenwasserstoffkette mit 2 bis 6 Kohlenstoffatomen darstellt.

3. Verfahren gemäß Anspruch I oder 2, dadurch **gekennzeichnet**, daß in den Verbindungen der Formel (I) R′ der Trimethylenrest: $-(CH_2)_3-$ oder eine Methyl-2-trimethylenkette:

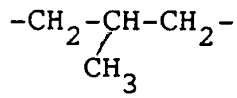

ist.

4. Verfahren gemäß einem der Ansprüche I bis 3, dadurch **gekennzeichnet**, daß in den Verbindungen der Formel (I) R einen Methylrest, R′ einen Trimethylenrest, p eine ganze Zahl von I bis 3 und q eine ganze Zahl von I00 bis I20 darstellen, wobei diese Verbindungen eine Molmassenzahl von 8000 bis I0000 aufweisen.

5. Schäumende Zusammensetzung zum Rasieren der Haut, dadurch **gekennzeichnet**, daß sie in einem wässrigen Milieu mindestens ein Polyorganosiloxan der Formel (I) gemäß der Ansprüche I bis 4 sowie ein schäumendes Mittel enthält.

6. Zusammensetzung gemäß Anspruch 5 dadurch **gekennzeichnet**, daß sie in der Form einer Creme, eines Gels, selbstschäumenden Gels, einer festen Seife oder eines Aerosolschaumes vorliegt.

7. Zusammensetzung nach einem der Ansprüche 5 und 6, dadurch **gekennzeichnet**, daß sie ein schäumendes Mittel aus einer $C_8$-$C_{20}$-Fettsäure, die mit einem alkalischen Mittel neutralisiert ist, oder ein anionisches synthetisches oberflächenaktives Mittel enthält.

8. Zusammensetzung gemäß Anspruch 7, dadurch **gekennzeichnet**, daß das anionische oberflächenaktive Mittel ausgewählt ist aus Alkalimetall- und Ammoniumsalzen von N-Acylglutamin-, Alkylthio-, Alkylsulfobernstein-, Alkylsulfoessigsäuren, N-Acylsarcosin, N-Acyl-N-methyltaurin, wobei die Alkyl- und Acylreste I4 bis 22 Kohlenstoffatome aufweisen.

9. Zusammenetzung gemäß Anspruch 7, dadurch **gekennzeichnet**, daß das schäumende Mittel eine Seife aus einer Mischung von $C_8$-$C_{20}$-Fettsäuren und $C_{16}$-$C_{20}$-Fettsäuren ist und die Mengenverhältnisse der $C_8$-$C_{20}$-Fettsäuren I0 bis 60 Gew.%, vorzugsweise I0 bis 25 Gew.%, bezogen auf Gesamtgewicht der Seifenmenge, und die Mengenverhältnisse der $C_{16}$-$C_{20}$-Fettsäuren 40 bis 90 Gew.%, vorzugsweise 75 bis 90 Gew.%, bezogen auf Gesamtgewicht der Seifenmenge, betragen.

I0. Zusammensetzung gemäß jedem der Ansprüche 5 bis 9, dadurch **gekennzeichnet**, daß sie I0 bis 85 Gew.% Wasser und 0,5 bis 80 Gew.% eines schäumenden Mittels gemäß der Ansprüche 7 bis 9 enthält.

II. Zusammensetzung gemäß jedem der Ansprüche 5 bis I0, dadurch **gekennzeichnet**, daß sie zusätzlich ein schäumendes polymer oder ein schäumendes nicht-ionisches, amphoteres oder ein von den in Anspruch 8 definierten unterschiedenes anionisches oberflächenaktives Mittel enthält.

I2. Zusammensetzung gemäß jedem der Ansprüche 5 bis II, dadurch **gekennzeichnet**, daß sie als Aerosol in der Gegenwart eines Treibmittelgases zubereitet ist, um im Augenblick der Freisetzung ein selbstschäumendes Gel oder einen Schaum zu bilden.

I3. Zusammensetzung gemäß Anspruch I2, dadurch **gekennzeichnet**, daß sie in Form eines Aerosolschaumes vorliegt und 75 bis 85 Gew.% Wasser, 0,5 bis I5 Gew.% eines schäumenden Mittels gemäß der Ansprüche 7 bis 9, 0,2 bis 3 Gew.% eines Organopolysiloxans der Formel (I) und 2 bis I5 Gew.%, vorzugsweise 3 bis I0 Gew.%, eines Treibmittelgases, bezogen auf Gesamtgewicht der Zusammensetzung, enthält.

I4. Zusammensetzung gemäß jedem der Ansprüche 5 bis I3, dadurch **gekennzeichnet**, daß sie zusätzlich Hydratisierungsmittel, Transparenzmittel, filmbildende Mittel, weichmachende Mittel, Anti-Akne-Mittel, antiseborrheische Mittel, Mineralöle, Fettalkohle, Polymere, Verdickungsmittel, Stabilisierungsmittel, Beruhigungsmittel, von den Seifen oder den in Anspruch 8 definierten unterschiedene anionische oberflächenaktive Mittel, nicht-ionische, amphotere oberflächenaktive Mittel oder deren Mischungen, Färbemittel, Konservierungsmittel, Antioxidantien und Pafüms enthält.

I5. Verfahren zum Rasieren der Haut, dadurch **gekennzeichnet**, daß man auf die Haut eine Zusammensetzung gemäß jedem der Ansprüche 5 bis I4 aufbringt, die Haut mit einem mechanischen Rasierer rasiert und mit Wasser spült.